# EUROPEAN PATENT SPECIFICATION

(11) **EP 0 996 447 B1**
(45) Date of publication and mention of the grant of the patent: **19.07.2006**
(21) Application number: 98922429.0
(22) Date of filing: 19.05.1998
(51) Int. Cl.: A61K 31/505, A61K 45/06, A61P 15/10

(54) **COMBINATION THERAPY FOR MODULATING THE HUMAN SEXUAL RESPONSE**
KOMBINATIONSTHERAPIE ZUR MODULATION DER HUMANEN SEXUALREAKTION
THERAPIE COMBINATOIRE PERMETTANT DE MODULER LA REPONSE SEXUELLE CHEZ UN PATIENT

(30) Priority: 19.05.1997 US 49947 P
(43) Date of publication of application: 03.05.2000
(73) Proprietor: ZONAGEN, INC., The Woodlands, TX 77380 (US)
(72) Inventor: PODOLSKI, Joseph, S., The Woodlands, TX 77381 (US)
(74) Representative: Walton, Seán Malcolm
(86) International application number: PCT/US1998/010230
(87) International publication number: WO 1998/052569

(56) References cited:
- EP-A- 0 268 146
- WO-A-95/05172
- CN-A- 1 113 757
- FR-A- 2 680 106
- US-A- 4 742 054
- US-A- 5 145 852
- HEDLUND ET AL: "Pharmacotherapy in Erectile Dysfunction Agents for Self-Injection Programs and Alternative Application Models" SCANDINAVIAN JOURNAL OF UROLOGY AND NEPHROLOGY, vol. 30, no. S179, 1996, pages 129-138, XP002076708
- KITZEN ET AL: "Potentiation of Phosphodiesterase Inhibitor Antithrombotic Activity with Alpha-2 Adrenergic Blockade" LIFE SCI., vol. 48, no. 7, 1991, pages PL31-PL35, XP002076709
- DATABASE WPI Week 9727 Derwent Publications Ltd., London, GB; AN 97-294834 XP002076710 & JP 09 110 674 (KAO CORPORATION) , 28 April 1997 & PATENT ABSTRACTS OF JAPAN vol. 97, no. 8, 29 August 1997 & JP 09 110674 A (KAO CORP), 28 April 1997

## Description

### FIELD OF THE INVENTION

The application is directed to the compositions useful in improving human sexual response on demand.

### BACKGROUND OF THE INVENTION

The human sexual response in both males and females results from a complex interplay of psychological, hormonal, and other physiological influences. One important aspect of human sexual response that is common to both men and women is the erectile response which itself results from an interplay between the autonomic nervous system, the endocrine system, and the circulatory system.

Failure of the erectile response is most common in men and is referred to as impotence. Impotence is the inability of a male to achieve or sustain a penile erection sufficient for vaginal penetration and intercourse. Numerous approaches have been taken in attempts to treat impotence. These approaches include the use of external or internally implanted penile prosthesis. (See, *e.g.,* U.S. Patent No. 5,065,744, to Zumanowsky). A variety of drugs and methods for administering drugs have also been used in attempts to treat impotence. For example, U.S. Patent No. 3,943,246 to Stürmer addresses treatment of impotence in men by buccal and peroral administration of daily doses of 300-1500 international units (I.U.) of oxytocin or daily divided doses of 150-250 I.U. of desamino-oxytocin. The patent states that the buccal administration of 100 I.U. three times a day for 14 days results in improvement of *impotentia erectionis* in 12 of the 16 patients treated.

U.S. Patent No. 4,530,920 to Nestor *et al*. suggests the possibility that administration of nonapeptide and decapeptide analogs of luteinizing hormone releasing hormone agonists may be useful in the induction or enhancement of sexual behavior or therapy for impotence or frigidity. Nestor *et al*. suggest numerous routes of administration of the analogs including buccal, sublingual, oral, parenteral (including subcutaneous, intramuscular, and intravenous administration), rectal, vaginal, and others.

U.S. Patent No. 4,139,617 to Grunwell *et al*. suggests buccal and other routes of administration of 19-oxygenated-androst-5-enes for the endocrine mediated enhancement of the libido in humans.

U.S. Patent No. 4,863,911 to Anderson *et al*. addresses methods for treating sexual dysfunction in mammals using a biooxidizable, blood-brain barrier penetrating estrogen derivative. One of the purported objects of the Anderson *et al*. invention is the treatment of "psychological impotence" in males. Test results showed that the drugs used in the study stimulated mounting behavior, intromission, and mount latency in castrated rats.

A number of publications have proposed the use of various vasodilators for the treatment of impotence in males. Attempts to utilize vasodilators for the treatment of impotence were prompted by the fact that a significant percentage of cases of impotence were noted to be vasculogenic, i.e. resulting from vascular insufficiency.

Voss *et al.,* U.S. Patent No. 4,801,587, issued January 31, 1989, addresses the use of an ointment containing a vasodilator and a carrier agent for topical application to the penis of impotent men. The Voss *et al.* patent also describes application of such an ointment into the urethra of the penis using a catheter as well as a multi-step regimen for applying a vasodilator to the skin of the penis. In addition, *Voss et al.* proposes the surgical removal of a portion of the fibrous sheath surrounding the corpora cavernosum, thereby facilitating the penetration of a vasodilator-containing ointment into the corpora cavernosum. Vasodilators suggested for use by Voss *et al*. include papaverine, hydralazine, sodium nitroprusside, phenoxybenzamine, and phentolamine. The Voss *et al*. patent, however, provides no information regarding the actual efficacy of the treatments proposed or the nature of the response to such treatments.

U.S. Patent No. 4,127,118 to Latorre describes treating male impotence by direct injection of the vasodilating drugs into the corpus cavernosum and the corpus spongiosum of the penis using a syringe and one or more hypodermic needles. More particularly, the Latorre patent proposes the intracavernosal and intraspongiosal injection of sympathomimetic amines such as nylidrin hydrochloride, adrenergic blocking agents such as tolazoline hydrochloride, and direct acting vasodilators such as isoxsuprine hydrochloride and nicotinyl alcohol.

Brindley, G.S. (*Br. J. Pharmac.* **87**:495-500, 1986) showed that, when injected directly into the corpus cavernosum using a hypodermic needle, certain smooth muscle relaxing drugs including phenoxybenzamine, phentolamine, thymoxamine, imipramine, verapamil, papaverine, and naftidrofuryl caused erection. This study noted that injection of an "appropriate dose of phenoxybenzamine or papaverine is followed by an unrelenting erection lasting for hours." Injection of the other drugs studied induced erections lasting from about 11 minutes to about 6.5 hours.

Zorgniotti *et al., J. Urol*. **133**:39-41 (1985) demonstrated that the intracavernosal injection of a combination of papaverine and phentolamine could result in an erection in otherwise impotent men. Similarly, Althof *et al. J. Sex Marital Ther*. **17(2):** 101-112 (1991) reported that intracavernosal injection of papaverine hydrochloride and phentolamine mesylate resulted in improved erectile ability in about 84% of patients injected. However, in that study the dropout rate was 57%, fibrotic nodules developed in 26% of the patients, 30% of the patients developed abnormal liver function values, and bruising occurred in 19% of the patients.

Other studies describing intracavernosal injection of drugs using hypodermic needles for the treatment of impotence include: Brindley, *J. Physiol*. **342**:24P (1983); Brindley, *Br. J. Psychiatr.* **143**:312-337 (1983); Virag, *Lancet* **ii**:978 (1982); and Virag, *et al., Angiology* **35**:79-87 (1984).

While intracavernosal injection may be useful for inducing erections in impotent men, the technique has numerous drawbacks. Obvious drawbacks include pain, risk of infection, inconvenience and interference with the spontaneity of the sex act. Priapism (prolonged and other painful erection) also appears to be a potential problem when using injection methods. See, *e.g.* Brindley, (1986). Another problem arising in some cases of intracavernosal injection involves the formation of fibrotic lesions in the penis. See, e.g., Corriere, *et al., J. Urol.* **140**:615-617 (1988) and Larsen, *et al., J. Urol.* **137**:292-293 (1987).

Phentolamine, which has been shown to have the potential to induce erection when injected intracavernosally, has also been administered orally to test its effects in men having non-specific erectile insufficiency (Gwinup, *Ann. Int. Med.* 15 July 1988, pp. 162-163). In that study, 16 patients ingested either a placebo or a 50 mg orally administered dose of phentolamine. Eleven of the 16 patients (including three placebo-treated patients) became tumescent, became more responsive to sexual stimulation, and were able to achieve an erection sufficient for vaginal penetration after waiting 1.5 hours to attempt intercourse.

Sonda *et al. J. Sex & Marital Ther.* **16(1):**15-21 reported that yohimbine ingestion resulted in subjective improvement in erectile ability in 38% of impotent men treated, but only 5% of the treated patients reported complete satisfaction.

Of interest to the background of the invention is the disclosure of Stanley *et al*., U.S. Patent No. 4,885,173, which addresses methods administering drugs having cardiovascular or renal vascular activity through use of a lollipop assertedly facilitating drug absorption through the mucosal tissues of the mouth, pharynx, and esophagus. The Stanley *et al*. patent proposes that a large number of lollipop-administered drugs may improve cardiovascular function including drugs exhibiting direct vasodilating effects, including calcium channel blockers, β-adrenergic blocking agents, serotonin receptor blocking agents, angina blocking agents, other anti-hypertensive agents, cardiac stimulating agents, and agents which improve renal vascular function.

U.S. Patent No. 5,059,603 to Rubin describes the topical administration to the penis of isoxsuprine and caffeine, and nitroglycerine and caffeine along with suitable carrier compounds for the treatment of impotence.

Giocco and Zorgniotti U.S. Patent No. 5,565,466 describes the transmucosal administration of a variety of vasodilators including phentolamine mesylate for modulating the human sexual response.

Sildenafil, (Viagra^{™}, Pfizer, Inc.) 5-[2-ethoxy-5-(4-methylpiperazine-1-ylsulfonyl)phenyl]-1-methyl-3-proplyl-6,7-dihydro-1-H-pyrazolo[4,3-d]pyrinudin-7-one has also been shown to be useful as an oral treatment for male erectile dysfunction. [Martel *et al., Sildenafil, Drugs of the Future,* **22**:138-143 (1997)]. See also, Boolell *et al., Int'l, J. Impot. Res*., **8**:47-52 (1996) and Boolell *et al., Br.J. Urol*., **78**:257-261 (1996). Sildenafil and related compounds are described in EP 0702555B1, is a phosphodiesterase V inhibitor and more particularly a cyclic GMP-specific phosphodiesterase inhibitor.

U.S. Patent No. 5,731,339 to Lowrey describes orally administrable fast acting compositions comprising vasodilators such as phentolamine mesylate phentolamine HCl and others which permit improvement in erectile ability or sexual responsiveness in both men and women.

Typically, the foregoing orally administrable compositions show success rates of less than 100% in treated populations. Results also show that the quality of the sexual response seen in patients, *i*.*e*., the relative rigidity of the erections achieved with for example Sildenafil is variable. [See, Boolell *et al., Br. J. Urol*., **78**:257-261 (1996).] Therefore, there continues to exist a need in the art for effective compositions and methods for modulating human sexual response and especially for enhancing erectile ability in individuals suffering from erectile dysfunction or for whom the quality of their sexual response is less than satisfactory. Ideally, such means would be convenient and simple to use, would not require a constant dosage regimen or even multiple doses to achieve desired results (*i*.*e*., would be available on demand), would be non-invasive, would allow a rapid and predictable capacity to improve sexual responsiveness, and would improve the quality of the sexual response.

### SUMMARY OF THE INVENTION

The present invention provides improved compositions and for improving human sexual response in both males and females by administering two or more pharmaceutically active agents to the circulation, the combination of which results in improved sexual responsiveness, for example, by improving blood flow to the genitalia by various physiological mechanisms.

An aspect of the present invention is directed to a combination of a first pharmaceutically active agent and a second pharmaceutically active agent wherein the first pharmaceutically active agent is selected from the group consisting of phentolamine, phentolamine mesylate and phentolamine hydrochloride, and wherein the second pharmaceutically active agent is apomorphine (Pentech Pharmaceuticals).

A preferred oral formulation comprises in combination, at least a first and a second pharmaceutically active agent in a rapidly dissolving orally administrable tablet, i.e. having a disintegration time of less than about twenty minutes. Preferred rapidly dissolving tablets have a disintegration time of from about 1 minute to about 10 minutes. Most preferred are rapidly dissolving tablets having the disintegration times of less than one minute. Preferred oral doses of phentolamine mesylate in the formulations of the present invention are preferably from about 5 mg to about 100 mg.

The present invention also relates to treating male impotence or modulating the male sexual response, by the compositions of the invention in an amount effective to allow increased blood flow to the penis wherein erectile ability is improved on demand.

Preferably, the amount of the pharmaceutically active agents used for treatment of male impotence or to otherwise improve sexual responsiveness in males or females is effective to improve responsiveness in from about 1 minute to about 60 minutes following administration of the agent.

The invention also relates to improving the sexual response in women (*i.e.*, excitation, plateau, and orgasmic phases of the female sexual response) on demand by oral administration of an effective amount of the compositions of the present invention.

The compositions of the present invention are also useful in preparation for sexual intercourse by virtue of the ability to improve the sexual response in both males and females in less than one hour after administration.

The pharmaceutically active agents may, in combination, be used for the manufacture of a medicament for oral administration to a male or female to improve, on demand, the sexual response in a human and, more particularly, to improve erectile ability in response to sexual stimulation.

It is also recognized that any of the pharmaceutically active agents useful in the practice of the invention may be used as a free base, pharmaceutically acceptable salts, hydrates, and other forms such as described with respect to phentolamine.

Numerous other advantages of the present invention will be apparent from the following detailed description of the invention including the accompanying examples and the appended claims.

### DETAILED DESCRIPTION

The human sexual response in both the male and female involves a complex interplay between endocrine, neurological and psychological components which result in certain physiological and anatomical responses in both men and women.

While there are obvious differences in the sexual response between men and women, one common aspect of the sexual response is the erectile response. The erectile response in both males and females is result of engorgement of the erectile tissues of the genitalia with blood in response to sexual stimulation (physical, psychological, or both).

The vasculature which serves erectile tissue in both men and women is similar. In particular, in both men and women, the arterial circulation to the erectile tissues of the genitalia derives from the common iliac artery which branches from abdominal aorta. The common iliac artery bifurcates into the internal and external iliac arteries. The internal pudic artery arises from the smaller of two terminal branches of the anterior trunk of the internal iliac artery. In the female, the internal pudic artery branches into the superficial perineal artery which supplies the labia pudenda. The internal pudic artery also branches into the artery of the bulb which supplies the bulbi vestibuli and the erectile tissue of the vagina. The artery of the corpus cavernosum, another branch of the internal pudic artery supplies the cavernous body of the clitoris. Still another branch of the internal pudic artery is the arteria dorsalis clitoridis which supplies the dorsum of the clitoris and terminates in the glans and membranous folds surrounding the clitoris which correspond to the prepuce of the male.

In the male, the internal pudic artery branches into the dorsal artery of the penis (which itself branches into a left and right branch) and the artery of the corpus cavernosum, all of which supply blood to the corpus cavernosum. The dorsal artery of the penis is analogous to the artery dorsalis clitoridis in the female, while the artery of the corpus cavernosum in the male is analogous to the artery of the same name in the female.

The male erectile response is regulated by the autonomic nervous system which controls blood flow to the penis via the interaction of peripheral nerves associated with the arterial vessels in and around the corpus cavernosum. In the non-aroused or non-erect state, the arteries serving the corpus cavernosum are maintained in a relatively constricted state, thereby limiting the blood flow to the corpus cavernosum. However, in the aroused state, the smooth muscles associated with the arteries relax under the influence of catecholamines and blood flow to the corpus cavernosum greatly increases, causing expansion and rigidity of the penis. Brindley, *supra* (1986) hypothesizes that smooth muscle contraction opens valves through which blood can flow from the corpus cavernosum into the extracavernosal veins. According to Brindley (1986), when the relevant smooth muscles relax, the valves close diminishing venous outflow from the corpus cavernosum. When accompanied by increased arterial blood flow into the corpus cavernosum, this results in engorgement of the corpus cavernosum and an erection.

The pre-orgasmic sexual response in females can be broken down into distinct phases. Both the excitement phase and the plateau phase involve vasodilation and engorgement (vasocongestion) of the genitalia with arterial blood in a manner analogous to the male erectile response.

The excitement phase of the female sexual response is characterized by vasocongestion in the walls of the vagina which leads to the transudation of vaginal fluids and vaginal lubrication. Further, the inner one-third of the vaginal barrel expands and the cervix and the body of the uterus become elevated. This is accompanied by the flattening and elevation of the labia majora and an increase in clitoral size. [Kolodny *et al., Textbook of Sexual Medicine,* Little and Brown, Boston, MA (1979)].

The plateau phase follows the excitement phase in the female sexual response and is characterized by prominent vasocongestion in the outer one-third of the vagina, causing a narrowing of the opening of the vagina and a retraction of the shaft and the glans of the clitoris against the symphysis pubis. These responses are also accompanied by a marked vasocongestion of the labia. [Kolodny, *supra* (1979)].

The vasocongestive aspects of the female sexual response are not restricted to the genitalia in that areolar engorgement also occurs, sometimes to the extent that it masks the antecedent nipple erection that usually accompanies the excitement phase.

The failure of the erectile response in men to the extent that vaginal penetration and sexual intercourse cannot be achieved is termed impotence. Impotence has numerous possible causes which can be broken down into several general classifications. Endocrine related impotence can result from primary gonadal failure, advanced diabetes mellitus, hypothyroidism, and as one of the secondary sequelae of pituitary adenoma, idiopathic or acquired hypogonadism, hyperprolactinemia and other endocrine abnormalities.

Chronic systemic illnesses such as cirrhosis, chronic renal failure, malignancies and other systemic diseases can also cause impotence. Neurogenic impotence arising in the central nervous system can be caused by temporal lobe disorders caused by trauma, epilepsy, neoplasms and stroke, intramedullary spinal lesions, paraplegia, and demyelinating disorders. Neurogenic causes of impotence arising in the peripheral nervous system include somatic or autonomic neuropathies, pelvic neoplasms, granulomas, trauma, and others. Urologic causes of impotence include complete prostatectomy, local trauma, neoplasms, Peyronie's disease, and others. In addition, as discussed above, a significant percentage of cases of impotence are vasculogenic in nature.

As many as half the cases of male impotence may be psychogenic because there is no readily-ascertainable organic cause for the disorder. Even when there appears to be an underlying organic cause of impotence, psychologic factors may play a role in the disorder.

The present invention is designed to modify the circulatory aspects of the erectile response on demand using vasoactive agents administered to the circulation using a rapidly dissolving orally administered formulation.

A number of pharmaceutically active agents may be used in the practice of the present invention based on demonstrated systemic efficacy as vasodilators or other demonstrated ability to either act as vasodilators and/or to improve sexual responsiveness. α-adrenergic antagonists include phentolamine, phentolamine hydrochloride, and phentolamine mesylate. Phentolamine mesylate is a preferred α-adrenergic agent vasodilator for use in the practice of the present invention.

Phentolamine can exist in unsolvated as well as solvated forms, including hydrated forms, *e*.*g*., hemi-hydrate. In general, the solvated forms, with pharmaceutically acceptable solvents such as water, ethanol and the like are equivalent to the unsolvated forms for purposes of the invention. Phentolamine can form pharmaceutically acceptable salts with organic and inorganic acids. Examples of suitable acids for salt formation are hydrohamic acids such as hydrochloric and hydrobromic; as well as other acids such as sulfuric, phosphoric, acetic, citric, oxalic, malonic, salicylic, malic, fumaric, succinic, ascorbic, maleic, methanesulfonic, toluenesulfonic, and other mineral and carboxylic acids known to those skilled in the art. The salts are prepared by contacting the free base form with a sufficient amount of the desired acid to produce a salt in the conventional manner. The free base forms may be regenerated by treating the salt with a suitable dilute aqueous base solution such as dilute aqueous sodium hydroxide, potassium carbonate, ammonia and sodium bicarbonate. The free base forms differ from their respective salt forms somewhat in certain physical properties, such as solubility in polar solvents, but the salts are otherwise equivalent to their respective free base form for purposes of this invention. Phentolamine can also form crystalline polymorph forms or crystalline forms thereof using suitable or conventional crystallization procedures.

The compositions of the present invention may exhibit additive effects, synergistic effects with respect to the actions of the individual components or each agent may potentiate the activity of the other. By using combinations of two or more pharmaceutically active agents, it is expected that lower doses of the individual agents may be used than would be necessary with a single agent.

The compositions also eliminate the need for continuous therapy by providing a single dose for rapidly improving erectile ability on demand.

According to one aspect of the present invention, the pharmaceutically active agents are administered orally in the form of a rapidly dissolving tablet formulation or a rapidly dissolving chewable tablet formulation, administrable formulations that permit the rapid introduction of the vasodilating substance to the circulation so as to improve erectile ability within a short time after administration of a single dose of the formulation. However, alternative dosage forms such as standard hard tablets are also included within the scope of the invention.

Compositions and methods of the present invention are thus more convenient and help minimize any side-effects that may arise as a result of continuous or daily administration of the drugs. In addition, methods of the present invention allow more spontaneity in sexual activity than allowed by other methods such as the intracavernosal injection of vasodilators, although certain combinations of pharmaceutically active agents may be administered by intracavernosal injection.

The examples set forth below are intended to be illustrative of the present invention and are not intended to limit the scope of the invention as set out in the appended claims.

Example 1 describes rapidly dissolving formulations for the oral administration of combinations of pharmaceutical bioavailability of phentolamine mesylate when administered using a rapidly dissolving oral formulation. Example 2 addresses the practice of the present invention in modulating the erectile response in females. Example 3 describes a study on the effect of phentolamine and a phosphodiesterase inhibitor on spontaneous erections in men.

### EXAMPLE 1

### RAPIDLY DISSOLVING FORMULATIONS FOR THE ORAL ADMINISTRATION OF VASODILATING AGENTS

One aspect of the present invention is directed to rapidly dissolving orally administered compositions for the rapid delivery of pharmaceutically active agents to the systemic circulation thereby allowing a rapid (on demand) onset of improved erectile ability in response to sexual stimulation. The composition comprises a combination of two or more pharmaceutically active agents, in accordance with claim 1.

Exemplary formulations of a rapidly dissolving tablet that includes phentolamine mesylate and Sildenafil (not within the present invention) are set out below.

**TABLE 1**

| | mg/tablet |
|---|---|
| Phentolamine Mesylate, USP | 40 |
| Sildenafil | 50 |
| Silicon Dioxide, NF | 8 |
| Stearic Acid, NF | 4 |
| Lactose, NF | 162 |
| Microcrystalline Cellulose, NF | 120 |
| Croscarmellose Sodium, NF | 16 |
| Total Tablet Weight | 400 |

The ingredients set out in Table 1 (and those used tablet formulations set out below) are finely divided and mixed thoroughly before being compression formed into tablets having a total weight of about 400 mg. Other methods of mixing and tablet formation will be readily apparent to those of skill in the art. Physical characteristics of the tablet prepared by this method include an average hardness of 10.7 Kp, an average thickness of about 0.20 inches and an average disintegration time of about 0.71 minutes.

As shown in Table 1, the disintegrant, croscarmellose sodium NF (available as Ac-Di-Sol®, from FMC Corporation) was used to accelerate the dissolution of the tablet although other disintegrants such as those described below may be used to achieve the same effect.

Tablets useful in the practice of the present invention may include other pharmaceutical excipients, pharmaceutically acceptable salts, carriers, and other substances well known in the art. Buffering agents, flavoring agents, and inert fillers such as lactose, sucrose, corn starch, binders such as acacia, cornstarch, or gelatin. Disintegrants such as potato starch and analgetic acid as well as other commercially available disintegrants including Explotab® sodium starch glycolate, Polyplasdone XL® crospovidone NF, Starch 1500® pregelatinized starch NF. Gissinger *et al*., "A Comparative Evaluation of the Properties of Some Tablet Disintegrants", *Drug Development and Industrial Pharnracy* **6(5)**:511-536 (1980) also describes other disintegrants and methods for measuring disintegration time of tablets. Disintegration times for the practice of the present invention are less than about 20 minutes, as measured in accordance with *European Pharmacopeia 1980*. More preferred are disintegration times of two minutes or less. Most preferred is a dissolution time of less than one minute. Preferred dissolution times may vary depending on the pharmacokinetic properties of the vasodilator agent itself.

Formulations useful in the practice of the present invention may vary so long as the formulation maintains the properties of rapid dissolution and improved bioavailability of the active ingredient or ingredients.

Another example of a rapidly disintegrating tablet formulation is described in U.S. patent no. 5,298,261 to Pebley *et al*., (the '26.1 patent). The '261 patent describes a rapidly dissolving tablet comprising a drug and a matrix network that has been vacuum-dried below the equilibrium freezing point of the matrix but above its collapse temperature. The matrix network set out in the '261 patent preferably includes a gum, a carbohydrate, and the drug. Preferred gums include acacia, guar, xanthin, carrageenan, or tragacanth. Preferred carbohydrates described in the '261 patent include mannitol, dextrose, sucrose, lactose, maltose, maltodextrin, or corn syrup solids.

Another rapidly dissolving drug carrier is described in U.S. patent no. 5,079,018 to Ecanow (the '018 patent). The '018 patent describes a readily dissolvable carrier that comprises a drug, an interim skeletal structure of a water soluble, hydratable gel or foam forming material, preferably a proteinaceous material.

Other illustrative formulations (not within the present invention) are set out below.

**Table 2**

| | mg/tablet |
|---|---|
| Phentolamine Mesylate, USP | 20 |
| Sildenafil (Viagra^{™}) | 50 |
| Silicon Dioxide, NF | 8 |
| Stearic Acid, NF | 4 |
| Lactose, NF | 182 |
| Microcrystalline Cellulose, NF | 120 |
| Croscarmellose Sodium, NF | 16 |
| Total Tablet Weight | 400 |

**Table 3**

| | mg/tablet |
|---|---|
| Phentolamine Mesylate, USP | 60 |
| Silicon Dioxide, NF | 8 |
| Stearic Acid, NF | 4 |
| Lactose, NF | 192 |
| Microcrystalline Cellulose, NF | 120 |
| Croscarmellose Sodium, NF | 16 |
| Total Tablet Weight | 400 |

After mixing of the ingredients, tablets are prepared by direct compression. Physical characteristics of the tablets prepared according to Tables 2 and 3 are very similar to those described for tablets prepared according to Table 1.

It is recognized that the phosphodiesterase inhibitors, dopaminergic agonists, and nitric oxide synthase enhancers may have bioavailability characteristics that differ from phentolamine mesylate. However, the foregoing information identified particular characteristics useful in optimizing the certain orally administrable formulations of the invention.

While the studies described above were conducted using a rapidly dissolving formulation (as a preferred embodiment), other formulations that allow rapid absorption of the combination of active agents and corresponding improvement in erectile ability are within the scope of the present invention. For example, a chewable tablet formulation is shown in Table 4.

**Table 4**

| | mg/tablet |
|---|---|
| Phentolamine Mesylate, USP | 40 |
| Sildenafil | 50 |
| Silicon Dioxide, NF | 12 |
| Stearic Acid, NF | 12 |
| Lactose, NF | 100 |
| Sweetrex | 298 |
| Aspartame | 40 |
| ProSweet | 8 |
| Peppermint Flavor #860-172 | 40 |
| Total Tablet Weight | 600 |

Appropriate doses of each pharmaceutically active agents for each route of administration are readily determined by those of ordinary skill in the art. By way of illustration, in order to determine the appropriate dose of each of the vasodilating agents of the present invention, one of ordinary skill in the art may use as a starting point, the usual published dosage of the vasodilator or phosphodiesterase inhibitor. The usual oral doses for commercially available vasodilators can be found in the Physician's Desk Reference published annually by Medical Economic Data, Montvale New Jersey, and in the available medical literature.

Sildenafil has been shown to be effective at various doses including 25mg and 50 mg (see Boolell *et al, Br. J. Urology, 78*:257-261 (1996)).

Using the established oral dosages as starting points, the optimal dosage for the specific route of administration can be determined by measuring baseline arterial blood flow in genital circulation of the patient prior to administration of the drug using a doppler ultrasound velocimeter as described in Gioco *et al*., U.S. Patent No. 5,565,466. Other methods such as thermography, plethysmography using, *e*.*g*., a RigiScan^{™} (Dacomed Corp., Minneapolis, MN), radiometric or scintigraphic methods, and other methods well known in the art may also be utilized to assess blood flow in the genitalia. See Boolell *et al., Br. J. Urol*., **78**:257-261 (1996) and Boolell *et al., Int. J. Impot. Res.,* **8**:47-52 (1996). Having established base line blood flow, various dosages of the respective vasodilators may be administered using the compositions encompassed by the present invention and their effect on blood flow may be measured. The magnitude of the increase in blood flow necessary to modulate or enhance the sexual response in humans may vary from individual to individual, but is readily determined as described in Zorgniotti *et al*. PCT/US94/09048. In addition, individual patients may be titrated with various dosages of the respective vasodilators until the optimum dosage is determined.

Vascular flow studies may also be coupled with assessments of sexual responsiveness as evidenced by the improvement of erectile ability as measured by the International Index of Erectile Function (Rosen *et al*., *Urology,* **49**:822-830 (1997); incorporated herein by reference).

### EXAMPLE 2

### MODULATION OF THE FEMALE SEXUAL RESPONSE

As discussed above, there are striking parallels between the vascular anatomy of male and female genitalia and in the sexual response facilitated by this vasculature. In both males and females, the erectile response takes place when under physical or psychological stimulation, blood flow to the genitalia increases by virtue of relaxation of smooth muscles in the arteries serving the genitalia.

The compositions of the present invention may be used to improve or enhance the sexual response in women whose sexual response is impaired as evidenced by diminished capacity to produce sufficient vaginal lubrication to facilitate comfortable penile penetration and by other symptoms of impaired sexual responsiveness that may be correlated with the erectile response.

As in the case of male sexual response, in the absence of any clinically diagnosed dysfunction in the female erectile response, the methods of the present invention may be used to enhance the normal female sexual response. The "on demand" aspect of the present invention will allow a more rapid response to sexual stimulation along with heightened sensation associated with excitement, plateau, and orgasmic stages of the female sexual response by virtue of the increased blood flow to the tissues.

In practice, enhancement of the female sexual response using the methods and compositions of the present invention are carried in much the same way as those described for males as described above.

The appropriate doses of the particular combinations of pharmaceutically active agents may be readily determined as described above. The female response may be measured using methods described in Masters, W.H. and Johnson, V.E., *Human Sexual Response,* Little, Brown, and Co., Boston (1966) which is incorporated herein by reference. Methods for measuring blood flow, including doppler ultrasonic velocimetry, thermography using for example an isothermal blood flow transducer, radioscintigraphic methods, photoplethysmography may be used as well as other methods well known in the art. In addition, measuring the contraction of the distal 1/3 as is characteristic of the plateau phase of female sexual response of the vagina may be measured using methods and equipment well known in the art including but not limited to strain gauges or other devices for measuring muscular contraction or muscle tension.

Enhanced sexual response may also be measured in a more subjective manner by simply asking the female subject to describe any change in sensation brought about by administration of the vasodilator by the methods of the present invention. Appropriate placebo controls should also be conducted to ascertain whether or not the effort is directly attributable to the administration of the vasodilator.

However, any of the pharmaceutically active agents included within the scope of the present invention may be used and their dosages determined as described above.

### REFERENCE EXAMPLE 3

### COMBINATION OF PHENTOLAMINE AND A PHOSPHODIESTERASE INHIBITOR

Papaverine, a known non-specific phosphodiesterase inhibitor, in combination with phentolamine mesylate was compounded in a rapidly dissolving formulation as set out in Table 5 except that papaverine at the doses indicated below was substituted for Sildenafil. Dosages were as follows:

**Table 5**

| | mg/tablet |
|---|---|
| Phentolamine Mesylate, (alone) | 40 |
| Papaverine (alone) | 150 |
| Phentolamine Mesylate plus | 40 |
| Papaverine | 75 |
| Phentolamine Mesylate plus | 40 |
| Papaverine | 150 |
| Phentolamine Mesylate plus | 40 |
| Papaverine | 300 |

The response rates with 40 mg phentolaime mesylate alone in an orally administrable tablet having a disintegration time of less than 20 minutes is typically 34% to 38% as measured by international index of erectile function (Rosen *et al., Urology,* **49**:822-830 (1997)).

However, the combination of 40 mg phentolamine in combination with 150 mg papaverine resulted in spontaneous erection in about 10% of the treated subjects. Subjects receiving 40 mg phentolamine in combination with 300 mg papaverine had an incidence of spontaneous erection of about 30%.

Those patients receiving phentolamine mesylate alone with no sexual stimulation did not exhibit spontaneous erections.

It was also noted that the combination of phentolamine and papaverine resulted in an increased incidence of sleepiness (70%), dizziness (30%), conjunctival hyperemia (40%), and chills (20%). Of all the subjects, 10% exhibited one or more of the following: constipation, sensation of erection, headache, nasal congestion, diarrhea, heartburn, or flushing.

## Claims

1. A composition comprising, in an orally administerable tablet, phentolamine or phentolamine mesylate or phentolamine hydrochloride, and apomorphine; and optionally a pharmaceutically acceptable carrier; the tablet having a disintegration time of less than about twenty minutes, as measured in accordance with European Pharmacopeia 1980.

2. The composition according to claim 1 wherein said composition comprises about 5mg to about 100mg of phentolamine mesylate.

3. The composition as defined in claim 1 comprising phentolamine mesylate and apomorphine wherein the tablet has a disintegration time of less than about ten minutes.

4. The composition of claim 3, wherein the tablet has a disintegration time of less than about one minute.

5. A composition as defined in any one of claims 1 to 4 for use in a method for improving human sexual response.

6. A composition as defined in any one of claims 1 to 4 for use in a method for treatment of male impotence.

7. Use of a composition as defined in any one of claims 1 to 4 in the manufacture of a medicament for improving human sexual response.

8. Use of a composition as defined in any one of claims 1 to 4 in the manufacture of a medicament for treatment of male impotence.

## Patentansprüche

1. Zusammensetzung, umfassend, in einer oral verabreichbaren Tablette, Phentolamin oder Phentolaminmesylat oder Phentolaminhydrochlorid und Apomorphin; und gegebenenfalls einen pharmazeutisch annehmbaren Träger; worin die Tablette eine Auflösungsdauer von weniger als etwa zwanzig Minuten, gemessen gemäß dem Europäischen Arzneibuch 1980, aufweist.

2. Zusammensetzung nach Anspruch 1, worin die Zusammensetzung etwa 5 mg bis etwa 100 mg Phentolaminmesylat umfasst.

3. Zusammensetzung nach Anspruch 1, umfassend Phentolaminmesylat und Apomorphin, worin die Tablette eine Auflösungsdauer von weniger als etwa 10 Minuten aufweist.

4. Zusammensetzung nach Anspruch 3, worin die Tablette eine Auflösungsdauer von weniger als etwa einer Minute aufweist.

5. Zusammensetzung nach einem der Ansprüche 1 bis 4 zur Verwendung in einem Verfahren zur Verbesserung der menschlichen sexuellen Reaktion.

6. Zusammensetzung nach einem der Ansprüche 1 bis 4 zur Verwendung in einem Verfahren zur Behandlung von männlicher Impotenz.

7. Verwendung einer Zusammensetzung nach einem der Ansprüche 1 bis 4 bei der Herstellung eines Medikaments zur Verbesserung der menschlichen sexuellen Reaktion.

8. Verwendung einer Zusammensetzung nach einem der Ansprüche 1 bis 4 bei der Herstellung eines Medikaments zur Behandlung von männlicher Impotenz.

## Revendications

1. Composition comprenant, dans un comprimé oralement administrable, de la phentolamine ou du mésylate ou de phentolamine ou du chlorhydrate de phentolamine et de l'apomorphine; et facultativement, un support pharmaceutiquement acceptable, le comprimé ayant une durée de désintégration de moins d'environ 20 minutes, en mesurant selon la Pharmacopée Européenne 1980.

2. Composition selon la revendication 1 où ladite composition comprend environ 5 mg à environ 100 mg de mésylate de phentolamine.

3. Composition telle que définie à la revendication 1, comprenant du mésylate de phentolamine et de l'apomorphine, où le comprimé a une durée de désintégration de moins d'environ dix minutes.

4. Composition de la revendication 3, où le comprimé a u ne durée de désintégration de moins d'environ une minute.

5. Composition telle que définie dans l'une quelconque des revendications 1 à 4 à utiliser dans une méthode pour améliorer la réponse sexuelle humaine.

6. Composition telle que définie dans l'une quelconque des revendications 1 à 4 à utiliser dans une méthode du traitement de l'impuissance masculine.

7. Utilisation d'une composition telle que définie dans l'une quelconque des revendications 1 à 4 dans la fabrication d'un médicament pour améliorer la réponse sexuelle humaine.

8. Utilisation d'une composition telle que définie dans l'une quelconque des revendications 1 à 4 dans la fabrication d'un médicament pour le traitement de l'impuissance de l'homme.
